# FASCICULE DE BREVET EUROPEEN

(11) **EP 3 500 321 B1**
(45) Date de publication et mention de la délivrance du brevet: **06.04.2022**
(21) Numéro de dépôt: 17737322.2
(22) Date de dépôt: 16.06.2017
(51) Int. Cl.: A61M 5/20, A61M 5/32, A61M 5/31

(54) **DISPOSITIF D'INJECTION AUTOMATIQUE À RETENUE DE SERINGUE AMÉLIORÉE**
AUTOMATISCHE INJEKTIONSVORRICHTUNG MIT VERBESSERTER SPRITZENHALTERUNG
AUTOMATIC INJECTION DEVICE WITH IMPROVED SYRINGE RETENTION

(30) Priorité: 16.08.2016 FR 1657764
(43) Date de publication de la demande: 26.06.2019
(73) Titulaire: Nemera La Verpilliere, 38290 La Verpilliere (FR)
(72) Inventeur: DUGAND, Pascal, 38780 Estrablin (FR); STAMP, Kevin, Chapeltown Sheffield S35 1AR (GB)
(74) Mandataire: LLR
(86) Numéro de dépôt international: PCT/FR2017/051565
(87) Numéro de publication internationale: WO 2018/033667

(56) Documents cités:
- WO-A1-2009/081103
- WO-A1-2009/081133
- WO-A1-2011/012849
- GB-A- 2 478 349
- US-A1- 2010 185 148

## Description

La présente invention concerne le domaine des dispositifs d'injection automatique de produits liquides, notamment pharmaceutiques.

Un dispositif d'injection automatique est généralement un dispositif médical permettant l'administration automatique d'un médicament liquide nécessitant une injection. Ces dispositifs permettent en particulier que des personnes, par exemple atteintes d'arthrite rhumatoïde, de sclérose en plaque, de diabète ou subissant un choc anaphylactique en cas d'allergie, s'injectent elles-mêmes leur dose de médicament de manière autonome.

Un exemple de dispositif d'injection automatique est décrit dans le document US 8 734 402. Le dispositif comprend une seringue d'injection qui contient le produit liquide à injecter et qui est munie d'une aiguille, et d'un support de seringue. Il suffit généralement d'appuyer brièvement le dispositif sur la peau du patient pour déclencher une pénétration de l'aiguille dans la peau, suivie d'une injection du produit liquide, puis de la rétractation de l'aiguille à l'intérieur du dispositif pour éviter de blesser une personne avec l'aiguille.

Plus précisément, le dispositif comprend un support de seringue configuré pour loger la seringue d'injection, de façon qu'elle soit montée fixe dans le support de seringue tout au long du fonctionnement du dispositif. Ce support de seringue est formé par deux demi-coques, semi-tubulaires, assemblées l'une à l'autre une fois la seringue d'injection disposée à l'intérieur, de façon à former une coque tubulaire autour de la seringue.

La seringue d'injection comprend une collerette à l'une de ses extrémités, et un capuchon de protection d'aiguille, par exemple un capuchon appelé RNS (pour "Rigid Needle Shield"), à son autre extrémité. Le diamètre du capuchon de protection est souvent supérieur à celui du corps de la seringue.

Le support de seringue dans lequel est logée la seringue a une forme générale de tube dont le diamètre intérieur est proche du diamètre extérieur de la partie centrale de la seringue. Il n'est pas toujours possible d'insérer la seringue dans le support de seringue après que les deux demi-coques qui le composent aient été assemblées, car la collerette et le capuchon sont plus larges que la partie centrale de la seringue et donc plus large que le diamètre intérieur du support de seringue. Or, généralement, l'assemblage de la seringue d'injection dans le dispositif d'injection automatique est réalisé par un laboratoire pharmaceutique et non par le fabricant du dispositif d'injection automatique, de sorte que toute simplification de l'assemblage de la seringue d'injection dans le dispositif d'injection automatique évite que le laboratoire pharmaceutique ait besoin de s'équiper en appareillages complexes.

Le document US 2010/0185148 décrit un dispositif d'injection automatique selon le préambule de la revendication 1.

L'invention a notamment pour but de fournir un dispositif d'injection automatique dans lequel l'assemblage de la seringue d'injection dans le dispositif d'injection automatique est simplifié.

A cet effet, l'invention a pour objet un dispositif d'injection automatique comprenant :
- un manchon d'extrémité
- un support de seringue monté coulissant par rapport au manchon d'extrémité, le support de seringue étant destiné à porter une seringue d'injection munie d'une aiguille d'injection et d'un capuchon de protection de cette aiguille d'injection,
caractérisé en ce qu'il comprend un diaphragme formé par au moins deux éléments de blocage montés coulissant sensiblement radialement dans le manchon d'extrémité entre une configuration permettant le passage du capuchon de protection de la seringue d'injection, dans laquelle des butées ménagées sur les éléments de blocage, destinées à coopérer avec un épaulement de la seringue d'injection, sont éloignées entres elles, et une configuration permettant l'immobilisation de la seringue d'injection, dans laquelle les butées des éléments de blocage sont rapprochées entre elles.

Ainsi, grâce au fait que le diaphragme peut être en configuration de passage du capuchon de protection, il est possible d'assembler la seringue d'injection portant le capuchon de protection dans la partie du dispositif d'injection en insérant la seringue d'injection axialement par une extrémité du support de seringue, le capuchon de protection étant inséré le premier. Il n'est donc plus nécessaire d'assembler le support de seringue après y avoir positionné la seringue d'injection et il est donc possible d'assembler les deux demi-coques composant le support de seringue avant d'y introduire la seringue d'injection ou d'utiliser un support de seringue qui n'est pas constitué de deux demi-coques.

En outre, lorsque la seringue d'injection est retenue par la collerette, les efforts axiaux liés à l'injection doivent être limités car les collerettes ne sont pas suffisamment résistantes pour supporter des efforts importants. Or, dans la présente invention le diaphragme peut être configuré pour retenir la seringue d'injection par un épaulement de cette seringue situé à la base de l'aiguille de la seringue d'injection. Dans cette configuration, les efforts axiaux sur la seringue d'injection ne sont pas transmis à la collerette située à l'extrémité du corps de seringue opposé à l'aiguille. Il devient ainsi envisageable d'utiliser des ressorts particulièrement raides, et donc d'injecter un produit présentant une viscosité élevée. En particulier, il est possible d'utiliser un ressort d'injection présentant une force en position comprimée de 20 Newton, voire 50 Newton ou encore 80 Newton ou plus. On comprendra qu'il est également envisageable d'utiliser une seringue d'injection ne comportant pas de collerette ou encore que la seringue d'injection prenne la forme d'une cartouche de réception du produit liquide.

Selon d'autres caractéristiques optionnelles correspondant à différents modes de réalisation du dispositif d'injection :
- les éléments de blocage comprennent des languettes flexibles anti-retour destiné à empêcher le retrait des éléments de blocage du dispositif d'injection en coopérant avec le manchon d'extrémité ;
- les éléments de blocage sont muni de surface saillantes destiné à frotter contre des surfaces complémentaires du manchon d'extrémité pour empêcher que les éléments de blocage ne se déplacent prématurément de leur configuration de passage vers leur configuration d'immobilisation ;
- le manchon d'extrémité est fixé à une extrémité distale d'un organe dit de contrôle de forme générale tubulaire, dans lequel coulisse le support de seringue, cet organe de contrôle étant monté télescopique dans un boitier extérieur, le mouvement relatif de l'organe de contrôle par rapport au boitier extérieur commandant l'activation du dispositif d'injection automatique ;
- les éléments de blocage sont munis de rampes destinées à coopérer avec le boitier extérieur pour se déplacer depuis leur configuration de passage vers leur configuration d'immobilisation de la seringue d'injection ;
- des pions sont montés coulissant dans l'organe de contrôle, ces pions étant contraints, par des moyens de rappel des pions, à appuyer sur des surfaces respectives des éléments de blocage dans lesquelles des creux sont ménagés de manière à créer des points durs retenant les éléments de blocage dans leurs configurations de passage ou d'immobilisation ;
- les moyens de rappel sont logés entre des surfaces respectives du support de seringue et des épaulements respectifs des pions de manière à rappeler élastiquement le support de seringue vers l'extrémité proximale de l'organe de contrôle ;
- l'organe de contrôle et le boitier extérieur comprennent des moyens de blocage anti-retour, empêchant le coulissement de l'organe de contrôle par rapport au boitier extérieur dans une direction ;
- les organes de blocage anti-retour comprennent une languette de l'organe de contrôle coopérant avec des crans ménagés dans le boitier extérieur ;
- les butées des éléments de blocage sont délimitées en partie par des surfaces convexes de ces éléments de blocage ;
- la seringue d'injection destinée à être portée par le support de seringue comporte un corps de seringue muni d'une extrémité distale portant l'aiguille d'injection, l'épaulement de la seringue d'injection contre lequel les butées des éléments de blocage sont destinées à coopérer délimitant l'extrémité proximale de l'aiguille d'injection.

L'invention sera mieux comprise à la lecture de la description qui va suivre donnée uniquement à titre d'exemple et faite en se référant aux dessins annexés, dans lesquels :
- la figure 1 est une vue en perspective d'un dispositif d'injection automatique selon un premier mode de réalisation de l'invention, dans une configuration initiale, précédant le fonctionnement de ce dispositif d'injection automatique;
- la figure 2 est une vue similaire à la figure 1, le dispositif d'injection automatique étant représenté sans un boitier extérieur;
- la figure 3 est une vue similaire à la figure 2, le dispositif d'injection étant représenté sans organe de contrôle ni manchon d'extrémité;
- la figures 4 est une vue en coupe respectivement selon le plan IV-IV de la figure 1 ;
- les figures 5 et 6 sont des vue de détails respectivement V et VI de la figure 4;
- les figures 7 et 8, 9 et 10 et 11 et 12 sont des vues de détails, semblables aux figures 5 et 6, du dispositif d'injection dans différentes configurations de fonctionnement, notamment :
   ∘ dans les figures 7 et 8, le dispositif d'injection est dans une configuration activée, avant injection,
   ∘ dans les figures 9 et 10, le dispositif d'injection est dans une configuration après injection, avant rétraction de l'aiguille d'injection,
   ∘ dans les figures 11 et 12, le dispositif d'injection est dans une configuration après rétraction de l'aiguille d'injection ;

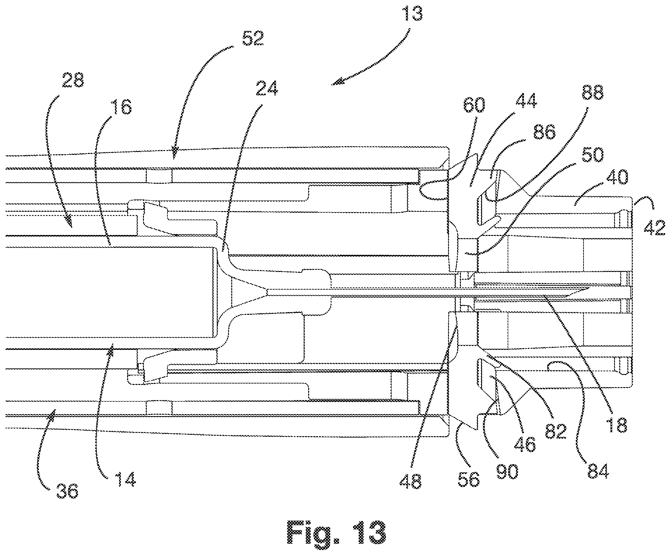

- la figure 13 est une vue en coupe de la partie avant d'un dispositif d'injection automatique selon un second mode de réalisation.

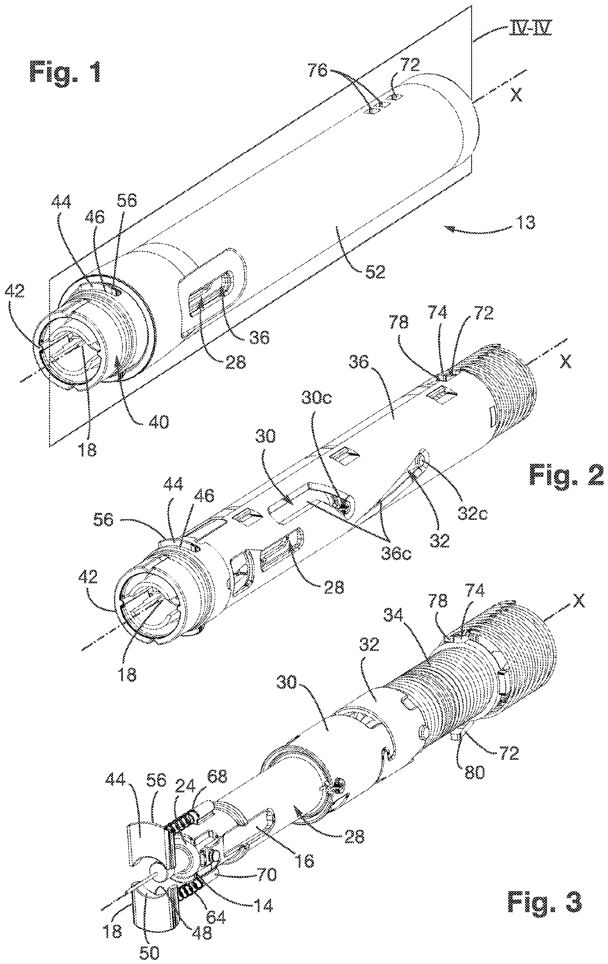

On a représenté sur les figures 1 à 12 un dispositif d'injection automatique 13 selon l'invention. Ce dispositif d'injection automatique 13 a une forme généralement de révolution d'axe X. Ce dispositif d'injection automatique 13 est destiné à être saisi manuellement par une extrémité, que l'on nommera par la suite extrémité proximale. L'extrémité opposée, que l'on nommera extrémité distale, est destinée à être appliquée contre la peau d'un patient, sur une zone d'injection.

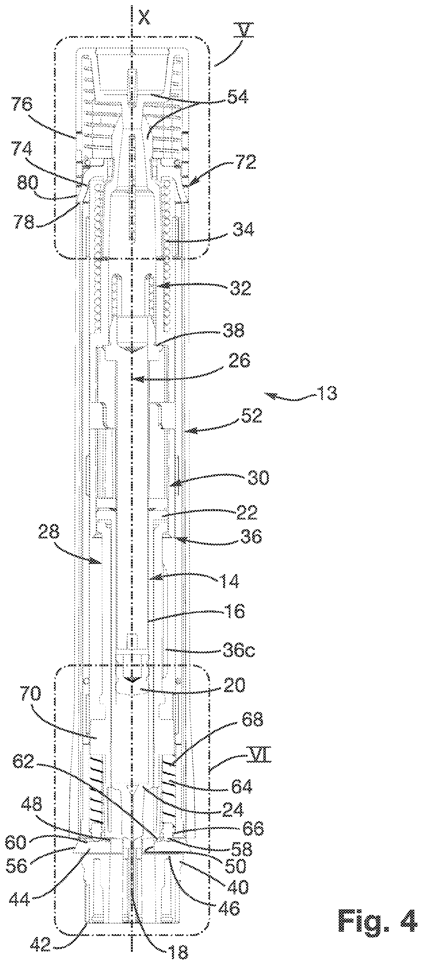

Le dispositif d'injection automatique 13 est destiné à être muni d'une seringue d'injection 14, visible notamment sur la figure 4. La seringue d'injection 14 est, dans ce premier mode de réalisation de l'invention, une seringue d'injection 14 en verre comprenant un corps de seringue 16 de forme généralement tubulaire d'axe X contenant un liquide pharmaceutique, éventuellement relativement visqueux, destiné à être injecté dans le corps du patient.

L'extrémité distale du corps de seringue 16 est munie d'une aiguille d'injection 18. Un piston 20 est monté coulissant dans le corps de seringue 16 de telle sorte que lorsque le piston 20 se déplace vers l'extrémité distale du corps de seringue 16, le liquide pharmaceutique contenu dans le corps de seringue 16 est expulsé à travers l'aiguille d'injection 18.

La seringue d'injection 14 est munie d'un capuchon classique (non représenté sur les figures) de protection de l'aiguille d'injection 18, recouvrant l'aiguille d'injection 18. Ce capuchon doit être enlevé avant l'utilisation du dispositif d'injection automatique 13.

A son extrémité proximale, le corps de seringue 16 est muni d'une collerette radiale 22. A son extrémité distale, le corps de seringue 16 comporte un épaulement distal 24.

Une tige de piston 26, visible sur la figure 4, est en contact avec une extrémité proximale du piston 20. Cette tige de piston 26 est destinée à pousser le piston 20 vers l'extrémité distale du corps de la seringue 16 lors de l'injection du produit pharmaceutique dans le corps du patient.

Le dispositif d'injection automatique 13 comprend un support de seringue 28, visible notamment sur les figures 3 et 4, destiné à porter la seringue d'injection 14. Le support de seringue 28 a une forme généralement tubulaire d'axe X comportant une ouverture à chaque extrémité distale et proximale.

Des organes de commande 30, 32 sont positionnés du coté proximal du support de seringue 28. Ces organes de commande 30, 32 sont destinés à coopérer entre eux et avec une le support de seringue 28 et la tige de piston 26. En coopérant, les organes de commande 30, 32 effectuent les étapes suivantes :
- d'abord, les organes de commandes 30, 32 poussent le support de seringue 28 de manière à ce que l'aiguille d'injection 18 soit insérée dans la peau du patient,
- puis les organes de commande 30, 32 poussent la tige de piston 26 et le piston 20 dans le corps de seringue 16 pour injecter le produit pharmaceutique,
- et enfin, les organes de commande 30, 32 libèrent axialement la tige de piston 26 et le support de seringue 28 afin que ceux-ci puissent reculer pour que l'aiguille d'injection 18 se rétracte, sorte de la peau du patient et se retrouve inaccessible.

Les organes de commande 30, 32 sont entrainés par un ressort d'injection 34 situé dans l'extrémité distale du dispositif d'injection automatique 13.

Le support de seringue 28 et les organes des commandes 30, 32 sont montés coulissant dans un organe de contrôle 36 du positionnement des organes de commande 30, 32. Des cames 30c, 32c ménagées sur les organes de commande 30, 32 coopérant avec des chemins de came 36c ménagés sur l'organe de contrôle 36, connus de l'état de l'art, pour permettre :
- aux organes de commandes 30, 32 de pivoter lorsqu'ils avancent dans l'organe de contrôle 36, et
- d'activer ou de désactiver des moyens d'embrayage 38 permettant le fonctionnement habituel du dispositif d'injection automatique 13.

L'organe de contrôle 36 a une forme générale tubulaire.

Un manchon d'extrémité 40 est fixé à l'extrémité distale de l'organe de contrôle 36. Le manchon d'extrémité 40 est muni d'une surface distale 42 destinée à venir en contact avec la peau du patient.

Deux éléments de blocage 44, visibles notamment sur la figure 3, sont montés coulissant sensiblement radialement dans deux fentes 46 ménagées dans le manchon d'extrémité 40 de manière à former un diaphragme. En variante, plus de deux éléments de blocage peuvent être utilisés. Chaque élément de blocage 44 est muni, à son extrémité la plus proche de l'axe X d'une butée 48 délimitée, en partie, par une surface convexe 50 de cet élément de blocage 44. Ces éléments de blocage 44 coulissent sensiblement radialement entre deux configurations :
- une configuration permettant le passage du capuchon de protection de la seringue d'injection 14, dans laquelle les butées 48 sont éloignées entre elles de manière à ce que l'écart entre les deux butées 48 soit suffisant pour laisser passer le capuchon de protection de la seringue d'injection 14,
- une configuration permettant l'immobilisation de la seringue d'injection 14, dans laquelle les butées 48 sont rapprochées entre elles de manière à ce que l'espace entre les deux butées 48 soit suffisamment faible pour retenir axialement la seringue d'injection 14 par son épaulement distal 24.

Ainsi, lorsque les éléments de blocage 44 sont dans leur position de passage du capuchon de protection de la seringue d'injection 14, il est possible de positionner la seringue d'injection 14 dans le dispositif d'injection automatique 13 sans que le capuchon de protection n'interfère avec les butées 48 des éléments de blocage 44. De même, lorsque les éléments de blocage 44 sont dans leur configuration d'immobilisation, la seringue d'injection 14 est retenue axialement lorsque l'aiguille d'injection 18 est insérée dans la peau du patient.

L'organe de contrôle 36 est monté télescopique à l'intérieur d'un boitier extérieur 52. On notera que l'organe de contrôle 36 est saillant du coté distal du boitier extérieur 52. Le boitier extérieur 52 est destiné à être saisi par son extrémité proximale par l'utilisateur. Le mouvement relatif du boitier extérieur 52 par rapport à l'organe de contrôle 36 commande l'activation du dispositif d'injection automatique 13. En configuration initiale du dispositif d'injection automatique 13, le boitier extérieur 52 et l'organe de contrôle 36 sont axialement éloignés, c'est-à-dire que l'organe de contrôle 36 est dans sa position la plus distale par rapport au boitier extérieur 52.

Pour activer le dispositif d'injection automatique 13, l'utilisateur applique l'extrémité distale du manchon d'extrémité 40 sur la zone d'injection et appuie brièvement le dispositif d'injection automatique 13 contre cette zone. Ainsi, l'organe de contrôle 36 se rétracte dans le boitier extérieur 52 et des moyens d'activation du dispositif 54, portés par l'un des organes de commande 32 et l'organe de contrôle 36, coopèrent de manière à activer le dispositif d'injection automatique 13.

Une rampe 56 est ménagée dans la partie la plus éloigné de l'axe X de chaque élément de blocage 44. Lors de l'activation du dispositif d'injection automatique 13, ces rampes 56 coopèrent avec l'extrémité distale du boitier extérieur 52 entrainant ainsi le déplacement des éléments de blocage 44 depuis leur position de libération vers leur position d'immobilisation de la seringue d'injection 14.

Deux pions 58 sont montés coulissants dans la partie distale de l'organe de contrôle 36. Chaque pion 58 coopère avec une surface proximale 60 d'un élément de blocage 44. Deux creux 62 sont ménagés dans chaque surface proximale 60 de manière à ce que les pions 58 soient logés dans les creux 62 lorsque les éléments de blocage 44 sont dans leur configuration de passage ou d'immobilisation. Des moyens de rappel 64, par exemple des ressorts de rappel ou tout autre élément équivalent, contraignent les pions 58 à appuyer sur les surfaces proximales 60 des éléments de blocage 44. Ainsi, lorsque les éléments de blocage 44 sont dans leur configuration de passage ou d'immobilisation, un effort est nécessaire pour initier le déplacement de ces éléments de blocage 44. Autrement dit, les pions 58, en coopérant avec les surfaces proximales 60 des éléments de blocage 44, créent des points durs retenant les éléments de blocage 44 dans leur configuration de passage ou d'immobilisation.

Les ressorts de rappel 64 des pions 58 sont logés entre des épaulements 66 ménagés sur les pions 58 et des surfaces 68 ménagées sur deux organes de guidage 70 du support de seringue 28. De cette manière, les ressorts de rappel 64 des pions 58 rappellent le support de seringue 28 vers l'extrémité proximale de l'organe de contrôle 36. Ainsi, lorsque la tige de piston 26 et le support de seringue 28 sont libérés axialement des organes de commandes 30, 32, les ressorts de rappel 64 des pions 58 déplacent le support de seringue 28 vers l'extrémité proximale de l'organe de contrôle 36 et ainsi, l'aiguille d'injection 18 se rétracte dans le dispositif d'injection automatique 13.

L'organe de contrôle 36 est muni de moyens de blocage anti-retour 72 à son extrémité proximale. Ces moyens de blocage anti-retour 72 comportent deux languettes 74 saillantes radialement de l'organe de contrôle 36. Ces languettes 74 sont destinées à coopérer avec des crans 76 ménagés dans le boitier extérieur 52. Chaque languette 74 peut coopérer avec trois crans 76 alignés axialement. Il y a donc trois paires de crans 76 réparties axialement, chaque paire de crans pouvant coopérer avec la paire de languettes 74. Les languettes 74 sont munies de butées distales 78 qui empêchent l'organe de contrôle 36 de coulisser vers l'extrémité distale du boitier extérieur 52 lorsqu'elles coopèrent avec les crans 76 du boitier extérieur 52. Par ailleurs, les languettes 74 sont inclinées de sorte à pouvoir se déformer élastiquement par coopération de surfaces 80 de ces languettes 74 avec les crans 76 du boitier extérieur 52. Ainsi, lorsque l'organe de contrôle 36 se déplace vers l'extrémité proximale du boitier extérieur 52, les languettes 74 se rétractent radialement et n'entravent pas ce mouvement.

De cette manière, l'organe de contrôle 36 et le boitier extérieur 52 peuvent être positionnés l'un par rapport à l'autre dans trois positions différentes, selon celle des trois paires de crans 76 du boitier extérieur 52 avec laquelle les languettes 74 de l'organe de contrôle 36 coopèrent. Ces trois configurations sont :
- une position initiale, dans laquelle le boitier extérieur 52 et l'organe de contrôle 36 sont axialement éloignés ; dans cette position les éléments de blocage 44 sont dans leur position de passage et le dispositif d'injection automatique 13 n'est pas activé ;
- une position intermédiaire, dans laquelle les éléments de blocage 44 sont dans leur position d'immobilisation de la seringue d'injection 14 mais le dispositif d'injection automatique 13 n'est pas activé ;
- une position d'activation, dans laquelle le boitier extérieur 52 et l'organe de contrôle 36 sont axialement rapprochés ; dans cette position les éléments de blocage 44 sont dans leur position d'immobilisation de la seringue d'injection 14 et le dispositif d'injection automatique 13 est activé.

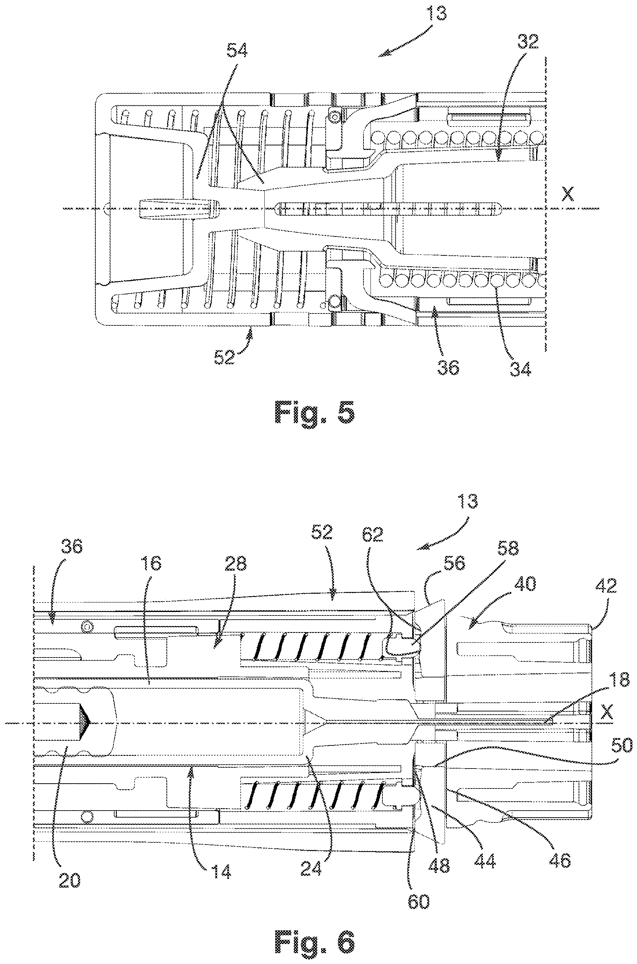

Les figures 5 à 12 représentent le dispositif d'injection automatique 13 dans plusieurs configurations correspondant à plusieurs étapes de son fonctionnement.

Les figures 5 et 6 représentent la configuration initiale du dispositif d'injection automatique 13, après que le capuchon de protection de l'aiguille d'injection 14 ait été retiré. Dans cette configuration, les éléments de blocage 44 sont dans leur configuration de passage et l'organe de contrôle 36 est positionné dans sa position initiale par rapport au boitier extérieur 52.

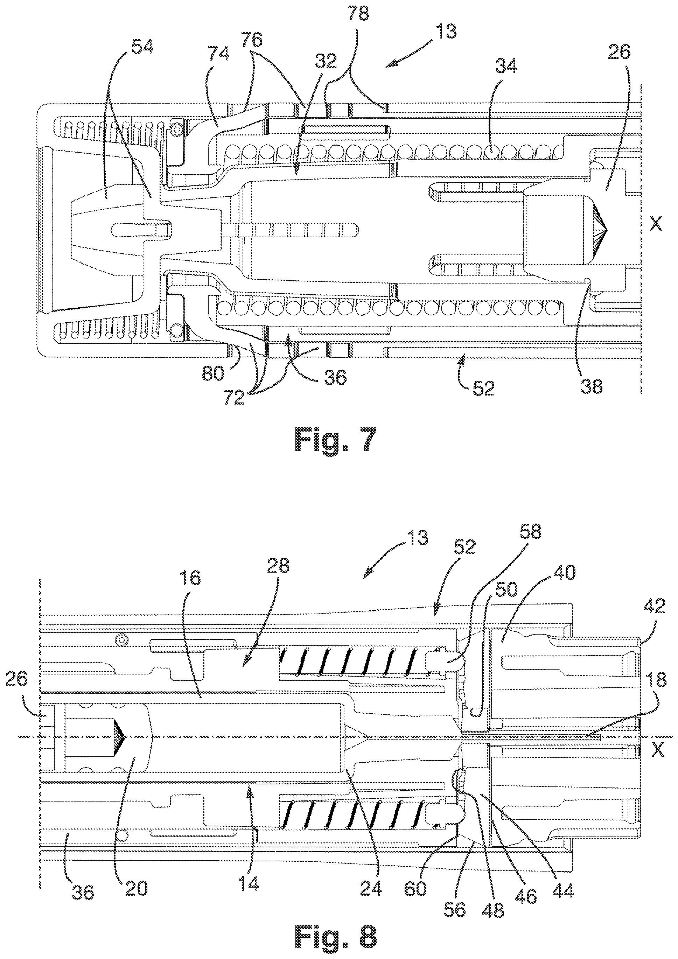

Les figures 7 et 8 représentent la configuration d'activation du dispositif d'injection automatique 13. Dans cette configuration, les éléments de blocage 44 sont dans leur configuration de blocage de la seringue d'injection 14. L'organe de contrôle 36 est dans sa position d'activation par rapport au boitier extérieur 52. Les languettes 74 de l'organe de contrôle 36 coopèrent avec les crans 76 les plus proximaux du boitier extérieur 52. Le boitier extérieur 52 et l'organe de contrôle 36 sont immobilisés l'un par rapport à l'autre dans la suite du fonctionnement du dispositif d'injection automatique 13.

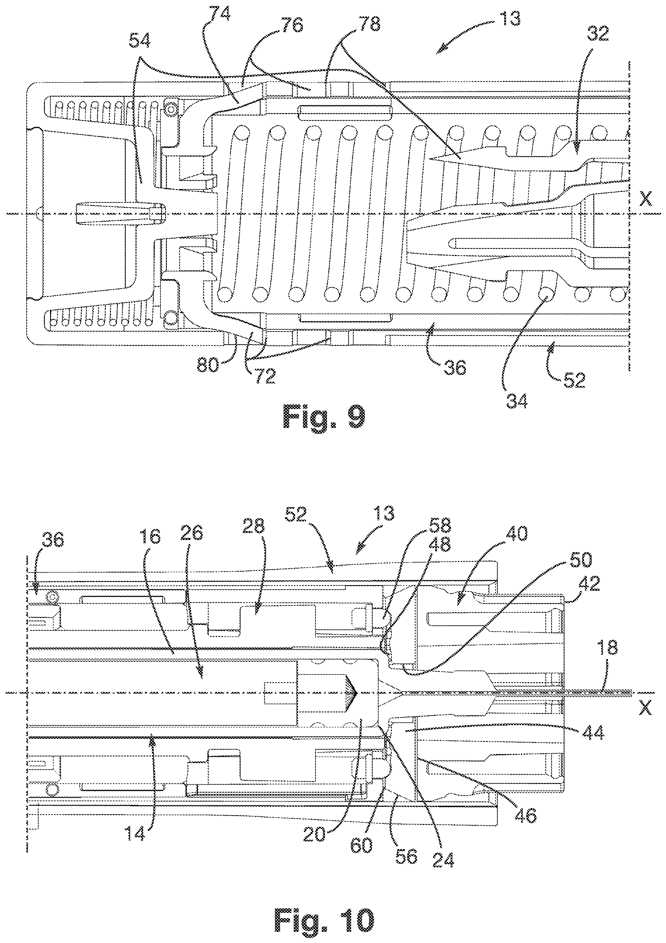

Les figures 9 et 10 représentent la configuration en fin d'injection du dispositif d'injection automatique 13. Dans cette configuration, l'épaulement distal de la seringue d'injection 14 est en appui contre les butées 48 des éléments de blocage 44. L'aiguille d'injection 18 est insérée dans la peau du patient et le piston 20 est en butée dans la partie distale du corps de seringue 16.

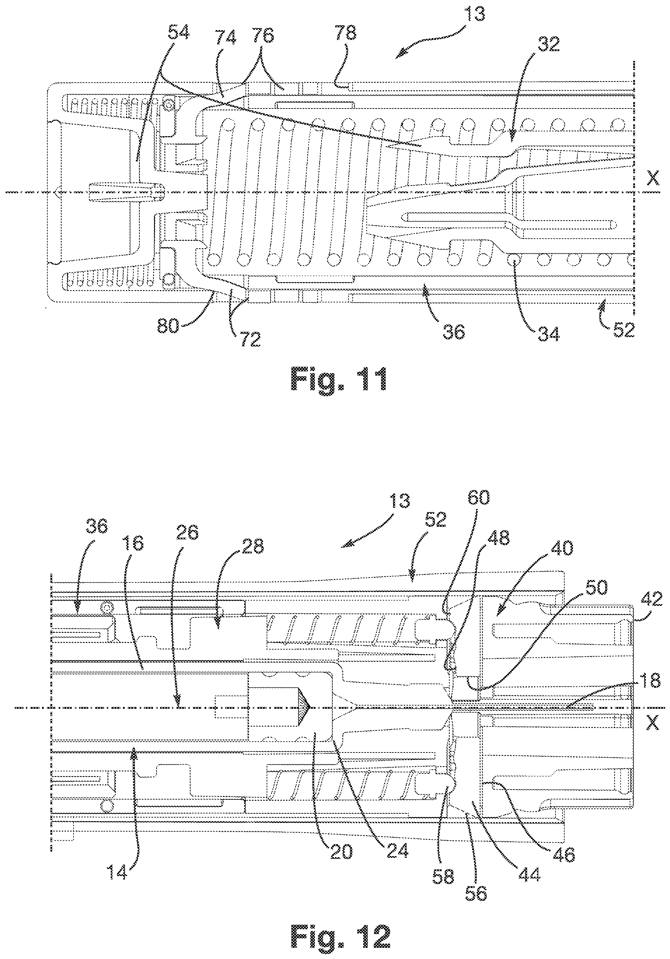

Les figures 11 et 12 représentent le dispositif d'injection automatique 13 après utilisation. Dans cette configuration, le support de seringue 28 est rappelé axialement vers la partie proximale de l'organe de contrôle 36. L'aiguille d'injection 18 est extraite de la peau du patient et n'est plus saillante par rapport au manchon d'extrémité 40.

La figure 13 représente un dispositif d'injection automatique 13 selon un second mode de réalisation de l'invention. Dans ce mode de réalisation, les éléments analogues au premier mode de réalisation sont désignés par des références identiques.

Dans ce mode de réalisation, le dispositif d'injection automatique 13 ne comprend pas de pions coopérant avec les surfaces proximales 60 des éléments de blocage 44. En remplacement de ces pions, les éléments de blocage 44 sont munis de languettes flexibles anti-retour 82 qui coopèrent avec une surface intérieure 84 du manchon d'extrémité 40 de manière à empêcher le retrait des éléments de blocage 44. Ces languettes flexibles anti-retour 82 empêchent les éléments de blocage 44 de se déplacer radialement vers l'extérieur, de cette manière, les éléments de blocage 44 ne peuvent pas être complètement séparés du reste du dispositif d'injection automatique 13.

Par ailleurs, chaque élément de blocage 44 est muni d'une saillie 86 comprenant une surface saillante 88 de l'élément de blocage 44. Cette surface saillante 88 est destinée à coopérer avec une surface complémentaire 90 du manchon d'extrémité 40 de manière à frotter contre cette surface complémentaire 90 lorsque l'élément de blocage 44 se déplace radialement vers l'axe X. De cette manière, l'élément de blocage 44 ne peut pas se déplacer prématurément, il ne peut se déplacer vers l'axe X que lorsqu'il est soumis à un effort radial, notamment quand la rampe 56 de l'élément de blocage 44 coopère avec le boitier extérieur 52.

L'invention n'est pas limitée aux modes de réalisation présentés et d'autres modes de réalisation apparaîtront clairement à l'homme du métier. Il est notamment possible d'utiliser plus de deux éléments de blocage ou un seul élément de blocage. De plus, la seringue d'injection peut être remplacée par une cartouche munie d'une aiguille.

## Revendications

1. Dispositif d'injection automatique (13) comprenant :
- un manchon d'extrémité (40)
- un support de seringue (28) monté coulissant par rapport au manchon d'extrémité (40), le support de seringue (28) étant destiné à porter une seringue d'injection (14) munie d'une aiguille d'injection (18) et d'un capuchon de protection de cette aiguille d'injection (18),
**caractérisé en ce qu'**il comprend un diaphragme formé par au moins deux éléments de blocage (44) montés coulissant sensiblement radialement dans le manchon d'extrémité (40) entre une configuration permettant le passage du capuchon de protection de la seringue d'injection (14), dans laquelle des butées (48) ménagées sur les éléments de blocage (44), destinées à coopérer avec un épaulement (24) de la seringue d'injection (14), sont éloignées entres elles de manière à ce que l'écart entre les butées (48) soit suffisant pour laisser passer le capuchon de protection de la seringue d'injection (14), et une configuration permettant l'immobilisation de la seringue d'injection (14), dans laquelle les butées (48) des éléments de blocage (44) sont rapprochées entre elles de manière à ce que l'espace entre les butées (48) soit suffisamment faible pour retenir axialement la seringue d'injection (14) par son épaulement distal (24).

2. Dispositif d'injection automatique (13) selon la revendication 1, dans lequel les éléments de blocage (44) comprennent des languettes flexibles anti-retour (82) destinées à empêcher le retrait des éléments de blocage (44) du dispositif d'injection automatique (13) en coopérant avec le manchon d'extrémité (40).

3. Dispositif d'injection automatique (13) selon la revendication 1 ou 2, dans lequel les éléments de blocage (44) sont munis de surfaces saillantes (88) destinées à frotter contre des surfaces complémentaires (90) du manchon d'extrémité (40) pour empêcher que les éléments de blocage (44) ne se déplacent prématurément de leur configuration de passage vers leur configuration d'immobilisation.

4. Dispositif d'injection automatique (13) selon l'une quelconque des revendications 1 à 3, dans lequel le manchon d'extrémité (40) est fixé à une extrémité distale d'un organe dit de contrôle (36) de forme générale tubulaire, dans lequel coulisse le support de seringue (28), cet organe de contrôle (36) étant monté télescopique dans un boitier extérieur (42), le mouvement relatif de l'organe de contrôle (36) par rapport au boitier extérieur (42) commandant l'activation du dispositif d'injection automatique (13).

5. Dispositif d'injection automatique (13) selon la revendication 4, dans lequel les éléments de blocage (44) sont munis de rampes (56) destinées à coopérer avec le boitier extérieur (42) pour se déplacer depuis leur configuration de passage vers leur configuration d'immobilisation de la seringue d'injection (14).

6. Dispositif d'injection automatique (13) selon la revendication 4 ou 5, dans lequel des pions (58) sont montés coulissant dans l'organe de contrôle (36), ces pions (58) étant contraints par des moyens de rappel (64) à appuyer sur des surfaces (60) respectives des éléments de blocage (44) dans lesquelles des creux (62) sont ménagés de manière à créer des points durs retenant les éléments de blocage (44) dans leurs configurations de passage ou d'immobilisation.

7. Dispositif d'injection automatique (13) selon la revendication 6, dans lequel les moyens de rappel sont logés entre des surfaces (68) respectives du support de seringue (28) et des épaulements (66) respectifs des pions (58) de manière à rappeler élastiquement le support de seringue (28) vers l'extrémité proximale de l'organe de contrôle (36).

8. Dispositif d'injection automatique (13) selon l'un quelconque des revendications 4 à 7, dans lequel l'organe de contrôle (36) et le boitier extérieur (42) comprennent des moyens de blocage anti-retour (72), empêchant le coulissement de l'organe de contrôle (36) par rapport au boitier extérieur (42) dans une direction.

9. Dispositif d'injection automatique (13) selon la revendication 8, dans lequel les organes de blocage anti-retour (72) comprennent une languette (74) de l'organe de contrôle (36) coopérant avec des crans (76) ménagés dans le boitier extérieur (42).

10. Dispositif d'injection automatique (13) selon l'une quelconque des revendications 1 à 9, dans lequel les butées (48) des éléments de blocage (44) sont délimitées en partie par des surfaces convexes (50) de ces éléments de blocage (44).

11. Dispositif d'injection automatique (13) selon l'une quelconque des revendications 1 à 10, dans lequel le support de seringue (28) porte une seringue d'injection (14) qui comporte un corps de seringue (16) muni d'une extrémité distale portant l'aiguille d'injection (18), l'épaulement (24) de la seringue d'injection (14) contre lequel les butées (48) des éléments de blocage (44) sont destinées à coopérer délimitant l'extrémité distal du corps de seringue (16).

## Patentansprüche

1. Automatische Injektionsvorrichtung (13) aufweisend:
- eine Endhülse (40)
- eine Spritzenhalterung (28), die in Bezug auf die Endhülse (40) gleitend montiert ist, wobei die Spritzenhalterung (28) dazu bestimmt ist, eine Injektionsspritze (14) zu tragen, die mit einer Injektionsnadel (18) und einer Schutzkappe für diese Injektionsnadel (18) versehen ist,
**dadurch gekennzeichnet, dass** sie eine Diaphragma aufweist, die aus mindestens zwei Sperrelementen (44) gebildet ist, die im Wesentlichen radial gleitend in der Endhülse (40) zwischen einer Einstellung montiert sind, die den Durchgang der Schutzkappe der Injektionsspritze (14) ermöglicht, in der auf den Sperrelementen (44) vorgesehene Anschläge (48), die dazu bestimmt sind, mit einer Schulter (24) der Injektionsspritze (14) zusammenzuwirken, voneinander beabstandet sind, so dass der Abstand zwischen den Anschlägen (48) ausreicht, um die Schutzkappe der Injektionsspritze (14) durchzulassen, und einer Einstellung, die eine Fixierung der Injektionsspritze (14) ermöglicht, bei der die Anschläge (48) der Sperrelemente (44) einander angenähert sind, so dass der Abstand zwischen den Anschlägen (48) klein genug ist, um die Injektionsspritze (14) mit ihrer distalen Schulter (24) axial festzuhalten.

2. Automatische Injektionsvorrichtung (13) nach Anspruch 1, wobei die Sperrelemente (44) flexible Rücklaufsperrzungen (82) aufweisen, die eingerichtet sind, das Entfernen der Sperrelemente (44) von der automatischen Injektionsvorrichtung (13) durch Zusammenwirken mit der Endhülse (40) zu verhindern.

3. Automatische Injektionsvorrichtung (13) nach Anspruch 1 oder 2, wobei die Sperrelemente (44) mit vorstehenden Oberflächen (88) versehen sind, die eingerichtet sind, gegen komplementäre Oberflächen (90) der Endhülse (40) zu reiben, um zu verhindern, dass sich die Sperrelemente (44) vorzeitig aus ihrer Durchgangseinstellung in ihre Fixiereinstellung bewegen.

4. Automatische Injektionsvorrichtung (13) nach einem der Ansprüche 1 bis 3, bei der die Endhülse (40) an einem distalen Ende eines sogenannten Steuerelements (36) von allgemein röhrenförmiger Gestalt befestigt ist, in dem der Spritzenträger (28) gleitet, wobei dieses Steuerelement (36) in einem Außengehäuse (42) teleskopisch montiert ist, wobei die Relativbewegung des Steuerelements (36) in Bezug auf das Außengehäuse (42) die Aktivierung der automatischen Injektionsvorrichtung (13) steuert.

5. Automatische Injektionsvorrichtung (13) nach Anspruch 4, bei der die Sperrelemente (44) mit Rampen (56) versehen sind, die eingerichtet sind, mit dem Außengehäuse (42) zusammenzuwirken, um sich von ihrer Durchgangseinstellung in ihre Einstellung zur Fixierung der Injektionsspritze (14) zu bewegen.

6. Automatische Injektionsvorrichtung (13) nach Anspruch 4 oder 5, bei der Stifte (58) gleitend in dem Steuerelement (36) angebracht sind, wobei diese Stifte (58) durch Rückstellmittel (64) gezwungen werden, auf jeweilige Oberflächen (60) der Sperrelemente (44) zu drücken, in denen Vertiefungen (62) ausgespart sind, um harte Punkte zu schaffen, die die Sperrelemente (44) in ihren Durchgangs- oder Fixiereinstellungen halten.

7. Automatische Injektionsvorrichtung (13) nach Anspruch 6, bei der die Rückstellmittel zwischen jeweiligen Oberflächen (68) der Spritzenhalterung (28) und jeweiligen Schultern (66) der Stifte (58) untergebracht sind, um die Spritzenhalterung (28) elastisch in Richtung des proximalen Endes des Steuerelements (36) zurückzustellen.

8. Automatische Injektionsvorrichtung (13) nach einem der Ansprüche 4 bis 7, wobei das Steuerelement (36) und das Außengehäuse (42) Rücklaufsperrmittel (72) aufweisen, die ein Verschieben des Steuerelements (36) relativ zum Außengehäuse (42) in einer Richtung verhindern.

9. Automatische Injektionsvorrichtung (13) nach Anspruch 8, bei der die Rücklaufsperrmittel (72) eine Zunge (74) des Steuerelements (36) aufweisen, die mit in dem Außengehäuse (42) ausgebildeten Rasten (76) zusammenwirkt.

10. Automatische Injektionsvorrichtung (13) nach einem der Ansprüche 1 bis 9, wobei die Anschläge (48) der Sperrelemente (44) teilweise durch konvexe Oberflächen (50) dieser Sperrelemente (44) begrenzt sind.

11. Automatische Injektionsvorrichtung (13) nach einem der Ansprüche 1 bis 10, bei der die Spritzenhalterung (28) eine Injektionsspritze (14) trägt, die einen Spritzenkörper (16) aufweist, der mit einem distalen Ende versehen ist, das die Injektionsnadel (18) trägt, wobei die Schulter (24) der Injektionsspritze (14), an der die Anschläge (48) der Sperrelemente (44) eingerichtet sind zusammenzuwirken, das distale Ende des Spritzenkörpers (16) begrenzt.

## Claims

1. Automatic injection device (13) comprising:
- an end sleeve (40)
- a syringe support (28) slidably mounted relative to the end sleeve (40), the syringe support (28) being intended to carry an injection syringe (14) provided with an injection needle (18) and with a cap for protecting this injection needle (18),
**characterised in that** it comprises a diaphragm formed by at least two locking elements (44) which are mounted so as to slide substantially radially in the end sleeve (40) between a passage configuration permitting the passage of the protecting cap of the injection syringe (14), in which configuration limit stops (48) formed on the locking elements (44), intended to cooperate with a shoulder (24) of the injection syringe (14), are moved away from each other so that the gap between the limit stops (48) is sufficient to permit the passage of the protecting cap of the injection syringe (14), and an immobilisation configuration permitting the immobilisation of the injection syringe (14), in which configuration the limit stops (48) of the locking elements (44) are moved towards each other so that the gap between the limit stops (48) is small enough to axially hold the injection syringe (14) by its distal shoulder (24).

2. Automatic injection device (13) according to claim 1, wherein the locking elements (44) comprise flexible non-return tabs (82) intended to prevent the locking elements (44) from being withdrawn from the automatic injection device (13) by cooperating with the end sleeve (40).

3. Automatic injection device (13) according to claim 1 or 2, wherein the locking elements (44) are provided with projecting surfaces (88) intended to rub against complementary surfaces (90) of the end sleeve (40) to prevent the locking elements (44) from moving prematurely from their passage configuration to their immobilisation configuration.

4. Automatic injection device (13) according to any one of claims 1 to 3, wherein the end sleeve (40) is fixed to a distal end of a so-called control member (36) of generally tubular shape, in which the syringe support (28) slides, this control member (36) being mounted telescopically in an outer casing (42), the relative movement of the control member (36) with respect to the outer casing (42) controlling actuation of the automatic injection device (13).

5. Automatic injection device (13) according to claim 4, wherein the locking elements (44) are provided with ramps (56) intended to cooperate with the outer casing (42) to move from their passage configuration to their immobilisation configuration immobilising the injection syringe (14).

6. Automatic injection device (13) according to claim 4 or 5, wherein pins (58) are mounted slidably in the control member (36), these pins (58) being forced by return means (64) to press on respective surfaces (60) of the locking elements (44) in which hollows (62) are formed so as to create hard points retaining the locking elements (44) in their passage or immobilisation configuration.

7. Automatic injection device (13) according to claim 6, wherein the return means are housed between respective surfaces (68) of the syringe support (28) and respective shoulders (66) of the pins (58) so as to elastically return the syringe support (28) to the proximal end of the control member (36).

8. Automatic injection device (13) according to any one of claims 4 to 7, wherein the control member (36) and the outer casing (42) comprise non-return locking means (72), preventing the control member (36) from sliding relative to the outer casing (42) in one direction.

9. Automatic injection device (13) according to claim 8, wherein the non-return locking means (72) comprise a tab (74) of the control member (36) cooperating with notches (76) formed in the outer casing (42).

10. Automatic injection device (13) according to any one of claims 1 to 9, wherein the limit stops (48) of the locking elements (44) are partly defined by convex surfaces (50) of these locking elements (44).

11. Automatic injection device (13) according to any one of claims 1 to 10, wherein the syringe support (28) carries an injection syringe (14) which comprises a syringe body (16) provided with a distal end carrying the injection needle (18), the shoulder (24) of the injection syringe (14) against which the limit stops (48) of the locking elements (44) are intended to cooperate delimiting the distal end of the syringe body (16).
